# EUROPEAN PATENT APPLICATION

(11) **EP 3 189 821 A1**
(43) Date of publication of application: **12.07.2017**
(21) Application number: 15837945.3
(22) Date of filing: 31.08.2015
(51) Int. Cl.: A61F 13/15, A47L 13/16

(54) **METHOD FOR MANUFACTURING WATER ABSORBENT ARTICLE AND ABSORBENT ARTICLE MANUFACTURED THEREBY**

(30) Priority: 01.09.2014 JP 2014177553
(71) Applicant: Yamada, Kikuo, Shinagawa-ku Tokyo 141-0022 (JP)
(72) Inventor: Yamada, Kikuo, Shinagawa-ku Tokyo 141-0022 (JP)
(74) Representative: Ishiguro, Masaoki
(86) International application number: PCT/JP2015/074753
(87) International publication number: WO 2016/035767

(57) **Abstract**

The purpose of the present invention is to provide a water absorbent article that has high water absorbency and high flexibility and, when used as a cleaning sheet, shows excellent ability to collect waste and dust even on a wet floor, etc. The method according to the present invention for manufacturing a water absorbent article (1) is characterized by comprising: an accumulating fiber process of accumulating a softness imparting layer (6) having absorbency on a surface sheet layer (5) having liquid permeability to form a sheet body (35); an embossing process of performing embossing on the sheet body (35) that is in a non-wet state to form a bulk portion formed from a plurality of uneven bodies in the sheet body (35); and a binder impregnating process of applying a binder to the sheet body (35) from outside after the embossing process and impregnating the binder Into the sheet body (35).

## Description

### Technical Field

The present invention relates to a method of manufacturing a water absorbent article that can be used for cleaning articles and a water absorbent article manufactured thereby.

### Background Art

A water absorbent article that can be used for cleaning articles includes multi-layered sheets of tissue paper adhered to one another through a water-soluble binder and embossed to form high bulky structure in the form of a plurality of protrusions and depressions. The embossed multi-layered sheets are then impregnated with an aqueous chemical. A cleaning article having such basic structure is disclosed in, for example, Patent Literature 1 below.

### Citation List

### Patent Literature

Patent Literature 1 : JP-U No. 2-103397

### Summary of Invention

### Technical Problem

Regarding the cleaning articles described in Patent Literature 1, a plurality of sheets of water-soluble paper are layered on the top of one another, between each two of which a water-soluble adhesive bonds. Projections and depressions are embossed into the sheets of water-soluble paper on the whole to form a towel element which is then impregnated with a sterilizing solution. However, the process of bonding between paper sheets by means of an adhesive requires time and manpower, increasing manufacturing cost.

Another way for manufacturing cleaning articles is through processes of layering a plurality of base paper sheets and then spraying a binder onto the base paper sheets, then embossing the base paper sheets and then impregnating them with aqueous chemicals. However, if the base paper sheets containing the binder are embossed, the base paper sheet adheres to an emboss roller, giving rise to the disadvantage that the embossed base paper sheet does not easily come off the roller.

To prevent the base paper sheet from adhering to the emboss roller, it is required to apply a releasing agent to the emboss roller or to apply a releasing agent to the base paper sheet, involving time, effort and difficulties.

The present invention has been made in view of the foregoing, and an object of the present invention is to provide a method for manufacturing a water absorbent article at low cost manufacture and in simple manufacturing processes without the disadvantage of adhesion to an emboss apparatus with a binder during an emboss process.

### Solution to Problem

The present invention is:
(1) A method for manufacturing an water absorbent article, including the steps of: an accumulating fiber process of accumulating a softness imparting layer having absorbency on a surface sheet layer having liquid permeability to form a sheet body; an embossing process of performing embossing on the sheet body that is in a non-wet state to form a bulk portion formed from a plurality of uneven bodies in the sheet body; and a binder impregnating process of applying a binder to the sheet body from outside after the embossing process and impregnating the binder into the sheet body.
(2) The method for manufacturing a water absorbent article according to (1), wherein the softness imparting layer is constituted by an aggregate of fibers, wherein the fibers exist densely in a compressed state in a region of a boundary face between the surface sheet layer and the softness imparting layer.
(3) The method for manufacturing a water absorbent article according to (1) or (2), wherein the softness imparting layer is formed from a crushed pulp of a material including crushed pulp.
(4) The method for manufacturing a water absorbent article according to (3), wherein a blending ratio of the crushed pulp in the softness imparting layer is 80% or more.
(5) The method for manufacturing a water absorbent article according to any one of (1) to (4), wherein the surface sheet layer is formed from a first surface sheet layer and a second surface sheet layer, wherein the softness imparting layer is formed between the first surface sheet layer and the second surface sheet layer.
(6) The method for manufacturing a water absorbent article according to any one of (1) to (5), wherein the binder is impregnated into the softness imparting layer in a region near joining face where the surface sheet layer and the softness imparting layer are joined.
(7) The method for manufacturing a water absorbent article according to any one of (1) to (6), wherein the binder is a cross-linked binder.
(8) The method for manufacturing a water absorbent article according to any one of (1) to (7), wherein the softness imparting layer has a bulk forming function.
(9) An absorbent article manufactured by the manufacturing method according to any one of (1) to (8).

### Advantageous Effects of Invention

According the present invention, a method for manufacturing a water absorbent article at low cost manufacture and in simple manufacturing processes without the disadvantage of adhesion to an emboss apparatus with a binder during an emboss process and a water absorbent article manufactured by the method can be provided.

### Brief Description of Drawings

[Fig. 1]
   Fig. 1 is a plan view illustrating a configuration of a first embodiment of a water absorbent article according to the present invention.
[Fig. 2]
   Fig. 2 is an enlarged perspective view of the water absorbent article according to the first embodiment.
[Fig. 3]
   Fig. 3 is a cross-sectional view taken along line A-A of Fig. 1.
[Fig. 4]
   Fig. 4 is a view to explain an example of a manufacturing process of the absorbent article.
[Fig. 5]
   Fig. 5 is a plan view illustrating a configuration of a second embodiment of the water absorbent article according to the present invention.
[Fig. 6]
   Fig. 6 is a cross-sectional view taken along line B-B of Fig. 5.
[Fig. 7]
   Fig. 7 is a cross-sectional view illustrating a configuration of a third embodiment of the water absorbent article according to the present invention.

### Description of Embodiments

A first embodiment of a water absorbent article 1 according to the present invention will be described with reference to Figs. 1 to 3. Fig. 1 is a plan view of a water absorbent article 1 according to the present invention, Fig. 2 is an enlarged perspective view of the water absorbent article, and Fig. 3 is a cross-sectional view taken along line A-A of Fig. 1.

As shown in Figs. 1 and 2, the water absorbent article 1 according to the present embodiment is configured as a sheet body of which a surface thereof is subjected to embossing and in which embosses 4 including a plurality of protrusions 2 and depressions 3 are formed. Note that shapes of the protrusions 2 and the depressions 3 of the emboss 4 can be determined as desired. For example, the shape of the emboss 4 may be circular, elliptical, triangular, rectangular, or rhombic; or a different shape such as, for example, a pattern/design or a corrugated shape in which the protrusions 2 and the depressions 3 having a linear shape are repeatedly formed. Moreover, these protrusions 2 and depressions 3 may be formed throughout an entire surface of the water absorbent article 1, or may be formed only in a portion of the water absorbent article 1. In the present embodiment, the case in which the water absorbent article 1 has the embosses 4 is described, but the embosses 4 may not necessarily be formed.

Moreover, the water absorbent article 1 of the present application may be water-disintegrable or non-water-disintegrable.

As shown in Fig. 3, the water absorbent article 1 of the present invention is formed from a surface sheet layer 5 having liquid permeability and a softness imparting layer 6 having absorbency. The surface sheet layer 5 is formed from a first surface sheet layer 7 and a second surface sheet layer 8, and the water absorbent article 1 is constituted by an accumulated-fiber body 1a in which the softness imparting layer 6 is accumulated between the first surface sheet layer 7 and the second surface sheet layer 8. Note that hereinafter, when the first surface sheet layer 7 and the second surface sheet layer 8 are described without distinction, the first surface sheet layer 7 and the second surface sheet layer 8 are referred to collectively as the surface sheet layer 5.

The surface sheet layer 5 and the softness imparting layer 6 are bonded through a binder that is impregnated into at least one layer of the surface sheet layer 5 and the softness imparting layer 6. In order to bond the surface sheet layer 5 and the softness imparting layer 6, the binder need to be impregnated into at least one layer of the boundary between the surface sheet layer 5 and the softness imparting layer 6. The "boundary between the surface sheet layer 5 and the softness imparting layer 6" includes a mode of between the first surface sheet layer 7 and the softness imparting layer 6, a mode of between the second surface sheet layer 8 and the softness imparting layer 6, a mode of between the first surface sheet layer 7 and the softness imparting layer 6, and a mode of between the second surface sheet layer 8 and the softness imparting layer 6. Particularly, the water absorbent article 1 is configured such that the binder is impregnated into the softness imparting layer 6 in any of a region near the boundary face 9a between the first surface sheet layer 7 and the softness imparting layer 6, a region near the boundary face 9b between the second surface sheet layer 8 and the softness imparting layer 6, or a region near the boundary face 9a and boundary face 9b, thus increasing the joining strength. Furthermore, preferably the binder is impregnated into both of the surface sheet layer 5 and the softness imparting layer 6 in the region, further increasing the joining strength between the surface sheet layer 5 and the softness imparting layer 6. Note that the boundary face 9a can be referred to as a joining face where the first surface sheet layer 7 and the softness imparting layer 6 are joined, and the boundary face 9b can be referred to as a joining face where the second surface sheet layer 8 and the softness imparting layer 6 are joined.

### Surface Sheet Layer

The surface sheet layer 5 is formed by a material having liquid permeability, but it is preferably formed using a paper material produced by a base paper sheet which is formed from pulp paper or a material including pulp as a principal raw material and subjected to various processing. For the paper material used for the surface sheet layer 5, the pulp preferably constitutes 30% or more. When the surface sheet layer 5 is formed from a paper material in which the pulp constitutes 30% or more, the softness of the water absorbent article 1 as a whole can be increased and production efficiency during manufacturing can be improved. Moreover, by increasing the proportion of the pulp, it is possible to facilitate the decomposition of the water absorbent article 1 after disposal in, for example, soil. Therefore, the environment burden can be further reduced and concern for the environment can be raised. For the paper material for forming the surface sheet layer 5, more preferably the pulp constitutes 50% or more, and furthermore preferably 80% or more. Note that when a plurality of surface sheet layers 5 are formed, the thickness and materials of the surface sheet layers 5 may be the same, or may be different.

Examples of raw material pulp include, for example, wood pulp, synthetic pulp, and waste paper pulp. The present invention can use toilet paper material as the raw material pulp. As the toilet paper material, for example, the raw material pulp constituted by blending, at predetermined proportions, softwood bleached kraft pulp obtained from softwood such as red pine, Yezo spruce, Sakhalin fir, Douglas fir, hemlock, and spruce; and hardwood bleached kraft pulp obtained from hardwood such as beech, oak, birch, eucalyptus, oak, poplar, and alder can be used. In present invention, the surface sheet layer 5 is not limited to natural fiber such as pulp, and regenerated fiber such as rayon can be also used. Although the blend of natural fiber and regenerated fiber can be used, natural fiber is preferably used. As natural fibers other than pulp, for example, kenaf, bamboo fibers, straw, cotton, cocoon filament, sugarcane and the like can be used. Note that, the paper material used in the surface sheet layer 5 may be water-disintegrable or may not be water-disintegrable. Moreover, the paper material used in the surface sheet layer 5 is not limited to the examples described above.

### Softness Imparting Layer

The softness imparting layer 6 is constituted by an aggregate of a plurality of fibers and has absorbency. The softness imparting layer 6 is formed from natural fiber such as pulp, regenerated fiber such as rayon, or a blend of natural fiber and regenerated fiber. As the natural fiber other than pulp, for example, kenaf, bamboo fibers, straw, cocoon, cocoon filament, sugarcane, and the like can be used. The softness imparting layer 6 is preferably formed such that a degree of density of the fibers in a thickness direction varies, and particularly it is preferably formed such that the fibers exist densely in a compressed state in a region of boundary face 9a between the first surface sheet layer 7 as the surface sheet layer 5 and the softness imparting layer 6, increasing the strength of the water absorbent article.

More preferably the softness imparting layer 6 is formed from the crushed pulp 11 or a material including the crushed pulp 11. The material used in the softness imparting layer 6 with the crushed pulp 11 includes pulp other than the crushed pulp, natural fiber other than pulp, and regenerated fiber such as rayon. Here, the term "crushed pulp 11" refers to a cotton-like material obtained by finely crushing the pulp material such as paper material that becomes the raw material, using a crusher. The raw material pulp of the crushed pulp 11 can include wood pulp, synthetic pulp, and waste paper pulp, as in the surface sheet layer 5, and the toilet paper material can be also used. As the toilet paper material, a blend of the softwood bleached kraft pulp and the hardwood bleached kraft pulp as in the surface sheet layer 5 can be used. However, from the perspective of manufacturing, a raw material pulp constituted from the softwood bleached kraft pulp is preferably used. Because the softwood bleached kraft pulp has longer fiber length than the hardwood bleached kraft pulp, when the softness imparting layer 6 is constituted using the crushed pulp 11 obtained from the softwood bleached kraft pulp, the degree of entangling of the fibers is increased, as a result, the strength is enhanced. Moreover, the volume of space between the fibers due to entanglement of the fibers is greater than that in the case where the hardwood bleached kraft pulp having shorter fiber length is used, and a degree of freedom for each of the fiber to move is increased, and thus the softness is improved. Note that the material of the pulp material used for forming the softness imparting layer 6 may be different from or the same as the material forming the surface sheet layer 5.

When the material used for the softness imparting layer 6 is formed from the crushed pulp 11 or the material including the crushed pulp 11 as a principal raw material, a blending proportion of the crushed pulp 11 in the material is preferably 30% or more, and more preferably 50% or more. Furthermore, even more preferably, the blending proportion of the crushed pulp 11 in the material is 80% or more. It is more preferable that the softness imparting layer 6 is formed from the crushed pulp by 100%. As the crushed pulp is formed to be cotton-like with the pulp material crushed, a larger number of spaces are formed between fibers than paper that is made through paper-making in which fibers are in a compressed state. When a large number of spaces are formed between fibers, a degree of freedom for each of the fibers constituting the softness imparting layer 6 to move can be increased. Therefore, when the crushed pulp 11 is blended at the above-mentioned proportion, a bulk forming function in the softness imparting layer 6 can be enhanced even at a lower basis weight, and the higher proportion of the crushed pulp 11 can enhance the bulk forming function even if the basis weight of the softness imparting layer 6 is reduced. Moreover, the large number of spaces which are formed between the fibers constituting the softness imparting layer 6 retain the higher water content, thus improving absorbency. As a result, the softness as a whole can be increased, and production efficiency during manufacturing can be improved. Note that the term "bulk forming function" is a function of increasing the bulk of the accumulated-fiber body 1a in which the surface sheet layer 5 and the softness imparting layer 6 are accumulated.

Note that the basis weight of the softness imparting layer 6 is preferably 80 g/m² or less, and more preferably 60 g/m² or less. By setting the basis weight of the softness imparting layer 6 to the range described above, manufacturing and packaging of the water absorbent article 1 can be facilitated, and the water absorbent article 1 can be formed having bulk that facilitates use by a user and packaging. Moreover, by setting the basis weight of the softness imparting layer to the range described above, there will be no too larger fiber density. As a result, an amount of the binder for joining between the fibers can be reduced. Therefore, a large amount of binder adhering to the surface of the surface sheet layer 5 and forming a film, and then decreasing the liquid permeability of the surface sheet layer 5 can be prevented, and absorbency of the water absorbent article 1 as a whole can be ensured.

### Binder

Any of various types of binders can be used, provided that the binder has a predetermined adhesive strength and can impart a predetermined strength to the surface sheet layer 5 and the softness imparting layer 6. Examples of the binder that can be used in the present invention include polysaccharide derivatives, natural polysaccharides, and synthetic polymers. Examples of the polysaccharide derivatives include carboxymethyl cellulose, carboxyethyl cellulose, carboxymethy starch or a salt thereof, starch, methyl cellulose, and ethyl cellulose. Examples of the natural polysaccharides include guar gum, tragacanth gum, xanthan gum, sodium alginate, carrageenan, gum arabic, gelatin, and casein. Moreover, examples of the synthetic polymer include polyvinyl alcohol, ethylene-vinyl acetate copolymer resin, polyvinyl alcohol derivative, polymers or copolymers of unsaturated carboxylic acid, or a salt thereof; and examples of the unsaturated carboxylic acids include acrylic acid, methacrylic acid, maleic anhydride, maleic acid, and fumaric acid. Of these, carboxymethyl cellulose and polyvinyl alcohol are particularly preferable.

When the binder is a cross-linked binder, it is preferable that the physical strength of the water absorbent article 1 is increased. The crosslinking agent for crosslinking the binder causes a crosslinking reaction with the binder 37 and forms the binder 37 into crosslinking structure. As a result, physical strength is increased.

When the binder having carboxyl group such as carboxymethyl cellulose is used, a polyvalent metal ion is preferably used as the crosslinking agent, and examples of the polyvalent metal ion include zinc, alkaline earth metals such as calcium and barium, and metal ions such as magnesium, aluminum, manganese, iron, cobalt, nickel, and copper. Specifically, ions of zinc, calcium, barium, magnesium, aluminum, iron, cobalt, nickel, and copper are advantageously used, which are preferable from the perspective of imparting a sufficient wet strength. The polyvalent metal ion as the crosslinking agent is used in the form of a water-soluble metal salt such as sulfate, chlorides, hydroxides, carbonates, and nitrates.

Moreover, when the polyvinyl alcohol is used as the binder, a titanium compound, a boron compound, a zirconium compound, a compound containing silicon, or the like can be used as the crosslinking agent, and one or a mixture of a plurality of these compounds can be also used as the crosslinking agent. Examples of the titanium compound include titanium lactate and titanium triethanol aminate; and examples of the boron compound include borax and boric acid. Moreover, examples of the zirconium compound include zirconium ammonium carbonate, and examples of the compound containing silicon include sodium silicate.

In the example described above, the case has been described where the water absorbent article is configured such that the surface sheet layers are on both surfaces of the softness imparting layer, but the water absorbent article may be configured such that the surface sheet layer is provided only on one surface of the softness imparting layer. Further, the surface sheet layers may be provided on both surfaces of the softness imparting layer, and a softness imparting layer may be further accumulated on one surface or both surfaces of the surface sheet layer. Furthermore, the surface sheet layer may be further provided on at least one surface of the softness imparting layer, and the number of the softness imparting layers and the surface sheet layers may be optionally adjusted.

### Manufacturing Method

Next, an example of a method for manufacturing the water absorbent article 1 according to the present invention will be described with reference to Fig. 4. Note that in the manufacturing method, "upstream side" refers to a side where the base paper sheet (described below) is supplied at a predetermined point in the manufacturing process, and "downstream" refers to a side where the supplied base paper sheet is discharged at a predetermined point in the manufacturing process.

In Fig. 4, reference sign 21 is a first feeding roll on which a first base paper sheet 24 for forming the first surface sheet layer 7 is wound, reference sign 22 is a second feeding roll on which a second base paper sheet 25 for forming the second surface sheet layer 8 is wound, and reference sign 23 is a third feed roll on which a pulp sheet 26 of crushed pulp raw material for forming the softness imparting layer 6 is wound. Moreover, the first base paper sheet 24, the second base paper sheet 25 and the pulp sheet 26 may employ a single layer structured sheet constituted by one layer of thin paper such as toilet paper or a multilayer structured sheet constituted by a plurality of layers of thin paper.

### Crushed Pulp Accumulating Process

The first base paper sheet 24 is fed by a first pinch roller 27 in a direction of the X arrow so as to be fed into a crusher 31. Additionally, the pulp sheet 26 is fed by a third pinch roller 29 in a direction of the Y arrow so as to be fed into the crusher 31.

The crusher 31 is provided with a crushing part 33 inside a housing 32, and an airflow forming mechanism F that draws an air in the housing 32 and forms an airflow. The pulp sheet 26 is crushed by the crushing part 33 upon being fed into the crusher 31.

Then, in the housing 32, the crushed pulp (crushed pulp 11) moves in a surface direction of the first base paper sheet 24 (in a direction B in the drawing) while the fibers are detangled or entanglement between the fibers is relaxed by the airflow formed by the airflow forming mechanism F, and is drawn and accumulated on the first base paper sheet 24 while being sequentially compressed.

Therefore, it is said that the fibers of the crushed pulp 11 exist densely in the most compressed state in the region of boundary face with the layer formed by the fibers of the crushed pulp 11 being accumulated on the surface of the first base paper sheet 24. Since the crushed pulp 11 exists densely in a compressed state in the boundary region between the first base paper sheet 24 forming the first surface sheet layer 7 and the layer of the crushed pulp 11 for forming the softness imparting layer 6, the fibers that exists densely and the fibers of the first base paper sheet 24 are densely joined by the binder 37, thus increasing the bonding strength with the first surface sheet layer 7 in the resulting absorbent article 1.

On the other hand, a density of the crushed pulp 11 in the boundary region between the layer of the crushed pulp 11 and the second base paper sheet 25 is more sparsely than the density in the boundary region between the first base paper sheet 24 and the layer of the crushed pulp 11 for forming the softness imparting layer 6, thereby weakening joining strength between the surface sheet layer 5 and the softness imparting layer 6 in the resulting absorbent article 1 and improving softness in the surface. Thus, the absorbent article 1 having both superior strength and softness can be obtained.

In the present embodiment, because the crushed pulp 11 is accumulated on the first base paper sheet 24 by the airflow as described above, the softness imparting layer 6 has softness compared with paper made by a paper-making process including a press process.

Moreover, for example, unlike a paper making process including a plurality of processes, the manufacturing method is simple because the crushed pulp has only to be crushed and accumulated.

Further, when the layer is formed through a paper making process, the presence of the press process causes the fiber-fiber to be crushed, but in the present embodiment, the fibers of the crushed pulp are only accumulated, so that there is a space between the fibers. As a result, the layer of the present embodiment can be bulkier at the same basis weight than that manufactured by a paper-making, and air permeability can be improved.

Furthermore, as the fiber-fiber is not crushed, the impregnation of the binder can be facilitated. As a result, the drying time in the manufacturing method can be shortened, and the manufacturing method can be efficient.

As described above, the first base paper sheet 24 on which the crushed pulp 11 has been accumulated in the housing 32 of the crusher 31 is discharged out of the crusher 31 while moving in the X direction.

### Second Base Paper Sheet Laminating Process

Then, on a downstream side of the crusher 31, the second base paper sheet 25 is laminated on the surface of the first base paper sheet 24 where the crushed pulp 11 is accumulated. The second base paper sheet 25 is fed in the Z direction by a second pinch roller 28, and is laminated on the first base paper sheet 24. At this point, the first base paper sheet 24, the crushed pulp 11 and the second base paper sheet 25 are sequentially laminated and accumulated to form a sheet body 35.

### Embossing Process

The sheet body 35 is passed through a pair of vertical emboss rollers 34, and is subjected to the embossing here. The emboss rollers 34 can employ a plurality of embossing protrusions (uneven bodies) on a peripheral surface of the rollers as conventionally known.

However, the emboss rollers 34 is not limited to this, and may be a combination of emboss rollers, in which the uneven bodies are projected, and flat rollers.

The material of emboss roller 34 may be a steel material or an elasticity material (such as a rubber material).

For example, uneven shaping by the embossing may be performed on only one side of the sheet body 35 from either one of the first base paper sheet 24 or the second base paper sheet 25, or may be performed on both front and back sides of the sheet body 35, that is, the first base paper sheet 24 side and the second base paper sheet 25 side.

The sheet body 35 is in a non-wet state at this time, and the embossing is performed on the sheet body 35 that is in a non-wet state.

Herein, the non-wet state does not include the mode in which moisture is applied to the sheet body 35 such as by blowing water on the sheet body 35. Ordinary, paper materials include moisture (water content) corresponding to conditions of air temperature and humidity, but the moisture (water content) is not water content that is actively supplied from outside. As such, even if such moisture (water content) is included, this corresponds to the non-wet state according to the present invention.

Accordingly, the percentage content of moisture (water content) included in the sheet body 35 varies depending the conditions of air temperature and humidity and regardless of the numeric value of the percentage content, it can be understood that this corresponds to the non-wet state according to the present invention.

The embossing is performed on the base paper sheet that is in an ordinary dry and non-wet state under the atmosphere, and thus there is no possibility of the sheet body 35 adhering to the emboss rollers 34, as there is in cases where the sheet body 35 is subjected to embossing in a wet state in which the sheet body 35 is impregnated with the binder. Therefore, there is no need to apply a releasing agent to the emboss rollers 34 or to apply a releasing agent to the sheet body 35.

The embossing is able to be performed without the emboss rollers 34 being heated, but the embossing may be performed with the emboss rollers 34 heated to a predetermined temperature. In the latter, a heating temperature for the emboss roller 34 preferably ranges from 60° C. to 150° C.

When the embossing is performed, the embosses 4 including the plurality of protrusions 2 and depressions 3 are formed in the sheet body 35 as shown in Figs. 1 and 2, and the plurality of embosses 4 forms a bulk portion 36. At this time, the bulky feel can be increased because the crushed pulp 11 exists between the first base paper sheet 24 and the second base paper sheet 25. As the crushed pulp 11 is cotton-like, when the softness imparting layer 6 is formed from the crushed pulp or a material including the crushed pulp, the softness imparting layer 6 can impart the bulky feel.

Herein, in the present invention, the embossing is performed on the sheet body 35 that is in a non-wet state, and thus, compared with the case of a wet state, the ductility during the process may not be necessarily excellent. In such a case, the sheet body 35 may not respond the load force of the embossing depending on an emboss depth, so that breaking of part of bonds between fibers may occur.

A small emboss depth is unlikely to cause such fiber-fiber bond breaking, but a large emboss depth is likely to cause the fiber-fiber bond breaking. For example, when the emboss depth ranges from 1 mm to 5 mm, the fiber-fiber bond breaking easily occurs. It is suggested that in present invention, the occurrence of fiber-fiber bond breaking is a desirable embodiment as described below, if anything.

In the present embodiment, in the method for manufacturing a water absorbent article, the base paper sheet to which water content is not supplied is used, and the base paper sheet which does not include a water-soluble binder and is in a non-wet state is subjected to embossing, and thus there is no possibility of the base paper sheet adhering to the emboss apparatus. Accordingly, there is no need to apply a releasing agent to the emboss apparatus, simplifying the embossing process and improving manufacturing efficiency. As a result, according to the present invention, the absorbent article can be easily manufactured and a reduction in manufacturing cost is achieved.

### Binder Impregnating Process

In the next process, the binder 37 such as carboxymethyl cellulose is applied to the sheet body 35 in which the bulk portion 36 is formed by the plurality of uneven bodies 4.

The binder 37 is applied by spraying a solution of the binder 37 from a nozzle of a spraying device onto the front surface and the back surface of the sheet body 35. Thus, the binder 37 is applied from the front and back (outer) sides of the sheet body 35 and impregnated into the sheet body 35.

In this case, in the present embodiment, the binder 37 is sprayed onto both the front and back surfaces of the sheet body 35 and impregnated into at least one of the surface sheet layer 5 and the softness imparting layer 6, that is, more specifically, the binder is impregnated into the first surface sheet layer 7, the second surface sheet layer 8 and the softness imparting layer 6. However, provided that the binder 37 capable of bonding the surface sheet layer 5 and the softness imparting layer 6 is impregnated into at least either of the surface sheet layer 5 and the softness imparting layer 6, the binder may be sprayed onto only the surface of one of the front and back sides of the sheet body 35.

A conventionally known spray nozzle may be selected as desired as the spray nozzle used for the spraying.

The means for supplying the binder 37 is not limited to the above-described spraying. For example, a technique of dropping the binder 37 onto the surface of the sheet body 35, and a technique of applying coating of the same, and the like may be adopted.

Moreover, binders other than carboxymethyl cellulose, polyvinyl alcohol, ethylene-vinyl acetate copolymer resin, and other substances may be used as the binder 37.

There are cases in which the binder 37 penetrates from one surface of the sheet body 35 to an opposing surface in the thickness direction and is impregnated throughout all layers in the thickness direction; and cases in which the binder 37 penetrates not to the opposing surface in the thickness direction, but only partway, that is, there are cases in which while the binder 37 is impregnated up to at least the boundary face between the first base paper sheet 24 the layer formed by the fibers of the crushed pulp 11 being accumulated thereon, and the boundary face between the second base paper sheet 25 and the layer formed by the fibers of the crushed pulp 11 being accumulated thereon, the binder 37 is not impregnated into the entirety of the sheet body 35, but rather into only a portion thereof.

This is the same for cases in which the solution of the binder 37 is applied from one side of the sheet body 35, and cases in which the solution of the binder 37 is applied from both sides of the sheet body 35. When a crosslinking agent of the binder is added as needed, the crosslinking agent can be applied simultaneously with the binder, but the present invention is not limited to this. The crosslinking agent may be applied and added at an arbitrary point in the manufacturing process.

By a supply of the binder as described above, the binder is impregnated into the sheet body 35. Here, the present embodiment includes the mode in which a part of fiber-fiber bond may break in the sheet body 35 in the embossing process.

When such fiber-fiber bond breaking occurs in the sheet body 35, a spot of the fiber-fiber bond breaking becomes a region that facilitates penetration of the binder, thereby increasing the speed of penetration of the binder as a whole.

Accordingly, the speed of penetration of the binder is greater than that in the case when the fiber-fiber bond breaking does not occur, enabling penetration of the binder with efficiency.

Moreover, when the binder flows into the spot of the fiber-fiber bond breaking, the fibers are bonded together through the binder, making the fiber-fiber bond tight.

Specifically, after the binder dries to a film, the strength of the binder film is greater than the strength of the fiber, thus achieving an enhancement in the strength of the sheet body 35. Furthermore, after cross-links are formed as described below, the strength of the binder film becomes further greater.

### Drying Process

The sheet body 35 impregnated with the binder 37 is transported to a dryer 38 and, here, subjected to drying. Hot-air drying, infrared drying, or a similar conventionally known method may be optionally selected and used as drying means. Regarding the drying by the dryer 38 of the sheet body 35 impregnated with the binder 37, the present invention is not limited to cases of drying using a single dryer 38, rather, a configuration is possible in which a plurality of dryers 38 are provided and the sheet body 35 is dried while being sequentially transported to each of the dryers 38.

The sheet body 35 that has been dried by the dryer 38 is then sequentially subjected to a folding process, a cutting process and the like as required. In the folding process, the sheet body 35 is guided to a folding machine 39 and is folded a predetermined number of times. After the folding process, the sheet body 35 is cut to predetermined dimensions and, thus, a folded body 40 of the sheet body 35 (the water absorbent article 1) is obtained.

Note that in the manufacturing method shown in Fig. 4, in the housing 32 of the crusher 31, the airflow forming mechanism F draws an air and forms an airflow to accumulate the crushed pulp 11 on the first base paper sheet 24 in a compressed state, but the method of accumulating the crushed pulp 11 on the first base paper sheet 24 is not limited to this. Moreover, an example was described in which embossing is performed on the sheet body 35 by the emboss roller 34, but the embossing may not be necessarily performed.

The water absorbent article 1 of the present invention can be used as raw materials of dry products such as tissue paper used in a non-wet state. Moreover, the water absorbent article 1, which has superior absorbency, can be used as raw materials for wet products such as cleaning goods for cleaning around toilets; a posterior wiping material; a body wipe for wiping the body; a facial sheet, makeup remover, or toning sheet for wiping a face; a wet tissue; a wet wipe or paper rag; and a wet floor wiping sheet which are used by impregnating an antimicrobial agent, a cleaning agent, or a detersive thereon.

### Second Embodiment

Next, a configuration of a second embodiment of the absorbent article according to the present invention will be described with reference to Figs. 5 and 6.

A water absorbent article 51 shown in Figs. 5 and 6 includes a surface sheet layer 54 formed from the first surface sheet layer 52 and the second surface sheet layer 53, and a softness imparting layer 55 is accumulated between the first surface sheet layer 52 and the second surface sheet layer 53. With the water absorbent article 51, a plurality of depressions 56 made by embossing are formed in an edge portion of the water absorbent article 51. The depression 56 is a concept including what is referred to as "spot processing" in the embossing.

Here, "spot processing" is a method for performing sealing of the first surface sheet layer 52 and the second surface sheet layer 53 by pressing the second surface sheet layer 53 (described below) in a spot-like manner toward the first surface sheet layer 52. In this case, the depression 56 is formed in the surface of the second surface sheet layer 53; the depression 56 is not formed in the surface of the first surface sheet layer 52, which is flat.

Even with the water absorbent article 51 formed in such a manner, the water absorbent article 51 having both strength and softness as a whole can be obtained. Moreover, with the water absorbent article 51, better feeling on the skin can be provided. Also, as in the absorbent article of the first embodiment, the water absorbent article 51 has superior absorbency.

### Third Embodiment

Next, a configuration of a third embodiment of the water absorbent article 1 according to the present invention will be described with reference to Fig. 7. A water absorbent article 61 shown in Fig. 7 has a two-layer configuration in which a softness imparting layer 63 is accumulated on a surface sheet layer 62. Note that specific configurations of the surface sheet layer 62 and the softness imparting layer 63 are the same as those described in the first embodiment. Therefore, description here is omitted.

Moreover, the apparatus for manufacturing the water absorbent article 1 according to the first embodiment includes the second feeding roll 22 on which the second base paper sheet 25 for forming the second surface sheet layer 8 is wound, but a manufacturing apparatus according to the present embodiment does not include the second feeding roll 22. Note that other configurations are the same as the first embodiment.

Furthermore, a method for manufacturing the absorbent article 1 does not include the second base paper sheet laminating process. Other processes are the same as the first embodiment.

Even with the water absorbent article 61 as shown in Fig. 7, by accumulating the softness imparting layer 63 on the surface sheet layer 62, the overall strength and softness of the water absorbent article 61 can be increased and the bulky feel of the water absorbent article 61 can be enhanced at a low basis weight. Thus, softness and strength as a whole can be imparted and a product with better feeling on the skin can be obtained. Also, the absorbent article according to the third embodiment has superior absorbency as those in the first and second embodiments.

As described above, embodiments of the absorbent article according to the present invention have been described in detail, but the description given above is an example of the absorbent article according to the present invention, and the present invention is not limited thereto. For example, it will be understood that the present invention can be used for absorbent articles such as paper diapers and sanitary articles. Moreover, in the embodiments described above, a formation was described in which embossing was performed on the surface sheet layer and the softness imparting layer, but a formation is possible in which embossing is not performed and, instead, edge portions of the surface sheet layer and the softness imparting layer are folded.

### Examples

### Example 1

In Example 1, a first base paper sheet formed from a paper material of a size of 100 mm × 100 mm, and a second base paper sheet formed from the same paper material as the first base paper sheet having a size of 100 mm × 100 mm and the same weight as the first base paper sheet were used. Moreover, a pulp sheet formed from pulp material was used. Moreover, a basis weight of the pulp sheet was 30 g/m².

Next, the pulp sheet was crushed and, the crushed pulp formed thereafter was accumulated on the first base paper sheet. Next, the first base paper sheet on which the crushed pulp was accumulated was fed out of the crusher, and the second base paper sheet was laminated on the crushed pulp. Thereafter, embossing was performed using emboss rollers and a bulk portion was formed by forming an uneven body. Note that hereinafter, the first base paper sheet in such a accumulated/laminated formation is referred to as the "first surface sheet" and the second base paper sheet in the formation is referred to as the "second surface sheet".

Next, a binder made from carboxymethyl cellulose was sprayed from a nozzle of a spraying device onto the front and back sides of the sheet body, and an aqueous solution of zinc sulfate as a crosslinking solution was sprayed thereon, thereby impregnating the binder and the crosslinking agent into the sheet body. Thereafter, the accumulated-fiber body was dried using a dryer.

Next, the aqueous solution of zinc sulfate as a crosslinking solution was sprayed on and impregnated into the dried accumulated-fiber body. Thereafter, the sheet body was folded by a folding machine and a folded absorbent article was obtained. A thickness of the obtained absorbent article was 0.17 mm.

### Peeling Test

A test was performed on the absorbent article obtained through the process described above in which the second surface sheet was peeled from the first surface sheet, and visual confirmation as to which, of the first surface sheet and the second surface sheet, the crushed pulp constituting the softness imparting layer was adhered to was carried out. Five sample pieces of the absorbent article obtained through the process described above were prepared and all of these samples were subjected to this peeling test. As a result, when the second surface sheet was peeled, an average value of a total weight of the first surface sheet and the crushed pulp adhered to the first surface sheet was 0.49 g, and an average value of a total weight of the second surface sheet and the crushed pulp adhered to the second surface sheet was 0.23 g. Thus, the weight of the first surface sheet and the crushed pulp adhered to the first surface sheet was greater than the weight of the second surface sheet and the crushed pulp adhered to the second surface sheet. A reason for this is thought to be because the fibers of the crushed pulp are accumulated on the first surface sheet by an airflow drawn and generated from a lower side of the first base paper sheet and, as such, the fibers of the crushed pulp exist densely in a compressed state in the region of the boundary face between the first surface sheet layer and the layer of the crushed pulp; and consequently, the joining strength due to the binder between the fibers of the first surface sheet layer and the fibers of the softness imparting layer is greater than the joining strength due to the binder between the fibers of the second surface sheet layer and the fibers of the softness imparting layer.

### Example 2

In Example 2, a first base paper sheet and a second base paper sheet of the same size and thickness as in Example 1 were used. Furthermore, a water absorbent article was obtained through the same process as in Example 1. Example 2 differed from Example 1 in that the basis weight of the pulp sheet was 20 g/m² and the thickness of the absorbent article was 0.13 mm.

### Peeling Test

As in Example 1, in Example 2, five sample pieces of the absorbent article were prepared and all of these samples were subjected to the peeling test. As a result, when the second surface sheet was peeled, an average value of a weight of the first surface sheet and the crushed pulp adhered to the first surface sheet was 0.37 g., and an average value of a weight of the second surface sheet and the crushed pulp adhered to the second surface sheet was 0.21 g. Thus the weight of the first surface sheet and the crushed pulp adhered to the first surface sheet was greater than the weight of the second surface sheet and the crushed pulp adhered to the second surface sheet. As expected, as in Example 1, the crushed pulp is accumulated on the first surface sheet by the airflow drawn and generated from a lower side of the first base paper sheet, therefore, the fibers of the crushed pulp exist densely in a compressed state at a position near the first base paper sheet. As a result, as the entanglement between the fiber of the crushed pulp and the fiber of the surface sheet layer increases, the area contacting the binder is larger. For these reasons, it is thought that the joining strength between the crushed pulp and the surface sheet layer increases and, more of the crushed pulp remains on the first surface sheet that has a higher density than on the second surface sheet and there is a change in weight.

### Reference Signs List

1, 51, 61 Water absorbent article
1 a Accumulated-fiber body
2 Protrusion
3, 56 Depression
4 Emboss
5, 54, 62 Surface sheet layer
6, 55, 63 Softness imparting layer
7, 52 First surface sheet layer
8, 53 Second surface sheet layer
9a, 9b Boundary face
11 Crushed pulp
24 First base paper sheet
25 Second base paper sheet
26 Pulp sheet
31 Crusher
32 Housing
33 Crushing part
34 Emboss roller
36 Bulk portion
37 Binder
38 Dryer
40 Folded body

## Claims

1. A method for manufacturing a water absorbent article, comprising the steps of:
an accumulating fiber process of accumulating a softness imparting layer having absorbency on a surface sheet layer having liquid permeability to form a sheet body;
an embossing process of performing embossing on the sheet body that is in a non-wet state to form a bulk portion formed from a plurality of uneven bodies in the sheet body; and
a binder impregnating process of applying a binder to the sheet body from outside after the embossing process and impregnating the binder into the sheet body.

2. The method for manufacturing a water absorbent article according to claim 1,
wherein the softness imparting layer is constituted by an aggregate of fibers,
wherein the fibers exist densely in a compressed state in a region of a boundary face between the surface sheet layer and the softness imparting layer.

3. The method for manufacturing a water absorbent article according to claim 1 or 2,
wherein the softness imparting layer is formed from crushed pulp or a material including crushed pulp.

4. The method for manufacturing a water absorbent article according to claim 3,
wherein a blending ratio of the crushed pulp in the softness imparting layer is 80% or more.

5. The method for manufacturing a water absorbent article according to any one of claims 1 to 4,
wherein the surface sheet layer is formed from a first surface sheet layer and a second surface sheet layer,
wherein the softness imparting layer is formed between the first surface sheet layer and the second surface sheet layer.

6. The method for manufacturing a water absorbent article according to any one of claims 1 to 5,
wherein the binder is impregnated into the softness imparting layer in a region near a joining face where the surface sheet layer and the softness imparting layer are joined.

7. The method for manufacturing a water absorbent article according to any one of claims 1 to 6,
wherein the binder is a cross-linked binder.

8. The method for manufacturing a water absorbent article according to any one of claims 1 to 7,
wherein the softness imparting layer has a bulk forming function.

9. A water absorbent article manufactured by the manufacturing method according to any one of claims 1 to 8.
